# EUROPEAN PATENT APPLICATION

(11) **EP 1 795 593 A1**
(43) Date of publication of application: **13.06.2007**
(21) Application number: 05774617.4
(22) Date of filing: 23.08.2005
(51) Int. Cl.: C12N 15/09, A61K 31/7088, A61K 48/00, A61P 31/12, A61P 31/18, C12Q 1/68

(54) **TARGET NUCLEIC ACID OF RETROVIRUS INTEGRATION**

(30) Priority: 24.08.2004 JP 2004244256
(71) Applicant: Kyoto University, Kyoto-shi, Kyoto 606-8501 (JP)
(72) Inventor: TSURUYAMA, Tatsuaki, c/o Graduate Sch. of Medicine, Kyoto-shi, Kyoto 606-8501 (JP)
(74) Representative: Weihs, Bruno Konrad
(86) International application number: PCT/JP2005/015255
(87) International publication number: WO 2006/022249

(57) **Abstract**

It is intended to provide a nucleic acid having such an activity as being available as a target of retrovirus integration; an antiviral agent containing this nucleic acid; a vector containing this nucleic acid; a method of treating a retroviral disease; and a method of conveniently and highly reproducibly testing the activity as a target of retrovirus integration. Namely, a nucleic acid which has a substantially palindromic sequence comprising a motif sequence: **5'-α₁-α₂-...-αₙ-X-βₙ- ...-β₂-β₁-3'** (wherein **α₁ to αₙ** represent each a sequence consisting of 4 to 7 consecutive bases in a sequence 5'-CACAGTG-3' or 5'-CACTGTG-3'; X represents an arbitrary sequence consisting of 0 to 10 bases; β₁ to βₙ represent each a sequence consisting of 4 to 7 bases and being substantially complementary in the reverse direction to one of the above-described sequences α₁ to αₙ; and n is an integer of 1 or above, provided that an arbitrary sequence consisting of from one to several bases may be sandwiched between each adjacent sequences in α₁ to αₙ and (β₁ to βₙ), an upstream sequence adjacent thereto and a downstream sequence adjacent thereto, and has an activity as a target of retrovirus integration.

## Description

### Technical Field

The present invention relates to a nucleic acid having an activity serving as a target of retrovirus integration into a host genome, a vector comprising the nucleic acid, an antiviral agent for prophylaxis or therapy of diseases attributable to a retrovirus, and a method of testing a nucleic acid for the activity serving as a target of retrovirus integration.

### Background Art

There are many diseases attributable to infection with a retrovirus, including AIDS, human adult T-cell leukemia, and so on. Retrovirus infection is initiated by adsorption of the virus onto a cell at a first stage. Then, a provirus genome of DNA is formed by a reverse transcriptase from the genomic RNA in the cell at a second stage. At a third stage, the DNA is integrated into a chromosomal DNA of the host, thereby completing infection. Given this completion of infection as a first step, a process leading to the onset of those diseases is initiated.

Accordingly, it is consideredthat a therapyorprophylaxis of diseases by a retrovirus could be carried out by inhibiting, as a target, the process of viral adsorption onto a cell, reverse transcription, or integration.

Antivirus agents being developed recently are those inhibiting the first or second stage mentioned above, while the development of prophylactic or therapeutic agents or methods for retrovirus diseases by inhibiting the integration stage is retarded. This is because elucidation of the mechanism of viral integration into a host chromosome is itself retarded.

Conventionally, it is known that in LTR, 6 base pairs including 2 base pairs (CA/GT) recognized by an integrase are essential as an integration signal sequence (ISS) for integration of retrovirus. However, there is no common sequence in the insertion site in host DNA, and thus integration of a viral gene in host DNA is considered to occur at random.

For example, Yoshinaga et al. developed an assay wherein an oligonucleotide of 21 base pairs synthesized by imitating the end of U5LTR was used both as a substrate (DNA to be integrated into a target) and as a target (DNA into which the substrate is to be inserted) to react with an integrase, thereby verifying whether *in vitro* integration occurred or not (Non-Patent Literature (Reference Literature) 1). As a result of this assay, however, it was found that the site of the target DNA, into which the substrate DNA is to be inserted, was indefinite.

As described above, the integration is considered to occur at a random site, and thus there had been neither a study on the mechanism of inhibition of specific integration utilizing structural specificity of the integration site nor development of inhibitor reagents based thereon. Therefore, only a few low-molecular compounds having an integrase inhibitory action have been reported as integration inhibitors.

Accordingly, antiretroviral agents and practical compositions or therapeutic methods for treating retroviral diseases based on the inhibition of integration have not been realized to date.

Prevention of a retrovirus being regenerated from a host cell carrying a latent virus in a proviral state and the regenerated virus re-infecting another normal cell (i.e., prevention of progress of the disease) cannot be attained by antiviral agents inhibiting the first or second stage. Thus, there is need for antiviral agents which can inhibit the third stage through integration inhibition.

Reference Literature 1: "AIDS - Hakken Kara Chiryo SaizensenMade" (AIDS - FromDiscoveryTo Therapeutic Forefront), authored by Shoichi Hatanaka, Kyoritsu Shuppan Co. Ltd. , January 1999, particularly pp. 41-47
Reference Literature 2: Hacein-Bey-Abina, S. et al., "LMO2-associated clonal T cell proliferation in two patients after gene therapy for SCID-X1," Science 302, 415-419 (2003)
Reference Literature 3: Wu, X., Li, Y., Crise, B., and Burgess, S.M., "Transcriptional start regions in the human genome are favored targets for MLV integration, " Science 300, 1749-1750 (2003)

### Disclosure of the Invention

An object of the present invention is to provide a nucleic acid having an activity capable of serving as a target of retrovirus integration, which, on the basis of the mechanism of insertion of a retrovirus into a host, can inhibit integration of a viral genome into a host genome at an initial stage of infection (i.e., prevention of infections or diseases) and can prevent regeneration of the virus from a host cell having a latent retrovirus in a proviral state and re-infection of another normal cell by the regenerated virus (i.e., prevention of progress of diseases), an antiviral agent comprising the nucleic acid, a vector comprising the nucleic acid, and a therapeutic method for retrovirus diseases. Another object of the present invention is to provide a method of easily, highly reproducibly and highly sensitively testing a nucleic acid for an activity as a target of retrovirus integration.

The present inventor found a commonality in retrovirus integration sites in mammals by analyzing a large number of the retrovirus integration sites for mouse lymphoma DNA and simultaneously analyzing human and mouse retrovirus integration sites recorded in public databases, and on the basis of this finding, the present invention was completed.

The present invention provides:
(1) a nucleic acid having an activity as a target of retrovirus integration, which has a substantial palindromic sequence comprising:
   a motif sequence: 5'-α₁-α₂-...-αₙ-x-βₙ-...-β₂-β₁-3' wherein α₁ to αₙ each represent a sequence consisting of contiguous 4 to 7 bases in 5'-CACAGTG-3' or 5'-CACTGTG-3', X represents an arbitrary sequence consisting of 0 to 10 bases, β₁ to βₙ each represent a sequence consisting of 4 to 7 bases substantially complementary, in the opposite direction, to the sequence α₁ to αₙ, respectively, n represents an integer of 1 or more, and an arbitrary sequence consisting of one to a few bases may be present between the adjacent sequences among the sequences α₁ to αₙ and β₁ to βₙ; and
   upstream and downstream sequences adjacent to the motif sequence, each of which has a length of 2 bases or more;
(2) the nucleic acid according to the above-mentioned (1), wherein each of the upstream and downstream sequences adjacent to the motif sequence consists of 4 or more bases;
(3) the nucleic acid according to the above-mentioned (1) or (2), wherein TCC or TTC is present in the upstream adjacent sequence, and GGA or GAA is present in the downstream adjacent sequence;
(4) the nucleic acid according to any of the above-mentioned (1) to (3), wherein the palindromic sequence is 36 to 100 bases in length;
(5) the nucleic acid according to any of the above-mentioned (1) to (4), which can bind to a retrovirus integrase;
(6) the nucleic acid according to any of the above-mentioned (1) to (5), wherein the motif sequence is a sequence set forth in any of SEQ ID NOS: 1, 2 and 9, or a sequence capable of hybridizing therewith under stringent conditions;
(7) the nucleic acid according to any of the above-mentioned (1) to (6), wherein the palindromic sequence is a sequence set forth in any of SEQ ID NOS: 3, 5 and 10, or a sequence capable of hybridizing therewith under stringent conditions;
(8) the nucleic acid according to any of the above-mentioned (1) to (7), which is in a form carried in a vector;
(9) an antiviral agent comprising the nucleic acid according to any of the above-mentioned (1) to (8);
(10) the antiviral agent according to the above-mentioned (9), which is a decoy-type drug or an antisense drug;
(11) an antiviral agent comprising, as a decoy, a nucleic acid with a substitution of one base in any of one or more sequences α and/or β in the motif sequence according to any of the above-mentioned (1) to (8);
(12) a method of testing a nucleic acid for an activity as a target of retrovirus integration, comprising the steps of:
   1) allowing double-stranded nucleic acids having at the 5'-side thereof protrusions of 2 bases, which nucleic acids derived respectively from the 5'- and 3' -ends of a retrovirus genome LTR sequence, or single-stranded nucleic acids capable of forming such nucleic acids, an integrase, and a cyclic nucleic acid containing a target sequence as a subject of examination to be simultaneously present; and
   2) detecting the presence or absence of the integration of the retrovirus genome-derived sequence into the target sequence;
(13) the method according to the above-mentioned (12), wherein in the step 1), the nucleic acid derived from the 5' -end and the nucleic acid derived from the 3' -end of the retrovirus genome LTR sequence are simultaneously combined with the other reaction components;
(14) the method according to the above-mentioned (12), wherein in the step (1), the nucleic acid derived from the 5' -end and the nucleic acid derived from the 3' -end of the retrovirus genome LTR sequence are reacted with the integrase separately to form integrase complexes, and then these integrase complexes are simultaneously combined and reacted with the cyclic nucleic acid;
(15) the method according to any of the above-mentioned (12) to (14), wherein the target sequence is at least 100 base pairs in length; and
(16) the method according to any of the above-mentioned (12) to (15), wherein the detection of the presence or absence of the integration is carried out by nucleic acid amplification and subsequent sequence analysis.

In the description of the present invention, the essential sequence present in the nucleic acid of the invention and represented by the following formula (1):

5' -α₁-α₂-...-αₙ-X-βₙ-...β₂-β₁-3'

wherein α₁ to αₙ each represent a sequence consisting of contiguous 4 to 7 bases in 5'-CACAGTG-3' or 5'-CACTGTG-3', X represents an arbitrary sequence consisting of 0 to 10 bases, β₁ to βₙ each represent a sequence consisting of 4 to 7 bases substantially complementary, in the opposite direction, to the sequence α₁ to αₙ, respectively, n represents an integer of 1 or more, and an arbitrary sequence consisting of one to a few bases may be present between the adjacent sequences among the sequences α₁ to αₙ and β₁ to βₙ, is referred to sometimes as a "motif sequence".

The term "an activity (serving) as a target of retrovirus integration" in the present invention refers to the property by which integration of a retrovirus genome can occur frequently, that is, the property which makes the nucleic acid liable to be a target of retrovirus genome integration. The nucleic acid with a high activity as a target of integration, when competes with another nucleic acid for binding to the integrase, binds to the integrase advantageously over another nucleic acid. Thus, as a result, the nucleic acid with a high activity as a target of integration has a high ability to inhibit the binding of another nucleic acid to the integrase and to inhibit the integration of a retrovirus genome into another nucleic acid.

When used with reference to the present invention, each term is used basically in usual meaning as used generally in the technical filed to which it pertains. The terms shown below particularly have the following meanings.

The "palindromic sequence" (or "palindromic structure") is used in general meaning. That is, this term refers to a sequence (or a structure) having the same sequence (or structure) upon reading from the 5' -end (or 3' -end) of complementary chains in a double-stranded nucleic acid. The nucleic acid having a palindromic sequence can complementarily bind therein with the palindromic sequence center as the turn-round to form a hairpin structure potentially. In this case, when a random sequence irrelevant to palindrome formation is inserted in the central position of the palindromic sequence, a loop structure is formed at the position of the random structure without forming base pairs. In other words, a palindromic sequence is a sequence capable of potentially forming a hairpin structure or a hairpin loop structure.

The term "substantially (substantial)" with respect to the complementarity in a palindromic sequence, a palindromic structure, a hairpin or hairpin loop structure, in formation of base pairs, and in sequences, refers to the situation in which complementarity of two involved sequences as a whole, or formation of base pairs or a specific structure as a whole is recognized, although the two sequences involved are not perfectly complementary, that is, the complementarity between the two sequences, or base-paring or formation of a specific structure, is imperfect due to the presence of a portion or portions with deletion or insertion of one or several bases (for example 2 to 4 bases) in one sequence. For example, a nucleic acid having a palindromic sequence consisting of "n" bases can be said to have a "substantial" palindromic sequence when the number of n is large to a certain degree, even if the nucleic acid contains one or a few bases, for example 2 to 4 bases, not forming base pairs upon formation of a hairpin structure.

According to the present invention, there can be provided a nucleic acid having an activity as a target of retrovirus integration. The nucleic acid of the invention utilizes fundamental properties common among various retroviruses and can thus be effective for essentially all retroviruses.

The nucleic acid of the invention can be used as an antiviral agent either alone or in combination with other ingredients. Particularly, the nucleic acid of the invention can be used in the form of an antisense drug or a decoy-type drug, by which it is possible to prevent a retrovirus from being regenerated from a latent proviral state in a host cell and to prevent the virus from re-infecting other normal cells (i.e., prevention of progress of diseases). That is, the antiviral agent of the invention enables prevention of retroviral re-infection of cells in the living body, and complete elimination of retroviruses from an infected individual when used in combination with another antiviral agent (an agent having an effect of killing an infected cell).

According to the method of the present invention, a nucleic acid of a specific sequence can be examined qualitatively or quantitatively for the activity as a target of retrovirus integration easily, highly reproducibly and highly sensitively. In the method of the invention, by using a nucleic acid of a target sequence in a cyclic form from the beginning, autonomous cyclization of the target sequence is prevented, thereby qualitative or quantitative judging of the binding of the target sequence to the integrase is rendered more evident than a conventional method. This effect is made more significant by using a relatively long target sequence.

According to the method of the present invention, by using both nucleic acids derived from the 5'- and 3'-ends of the retrovirus genome LTR sequence as a substrate nucleic acid, an integrase dimer or tetramer is formed between an integrase and the four single-stranded chains contained in two kinds of double-stranded chains in the substrate nucleic acid, which is advantageous for the integration reaction. Thus, the detection sensitivity of the integration reaction, as compared with conventional methods using either the 5'- or 3'-side, is improved.

### Brief Description of the Drawings

Fig. 1 is an illustration showing a hairpin structure formed potentially by a palindromic sequence set forth in SEQ ID NO: 3.
Fig. 2 is a diagrammatic illustration showing one mode of the method of the present invention.
Fig. 3 shows the results obtained in the Examples of the method of the invention by means of the target sequence used in the Examples and the presence or frequency of insertion into each position of the sequence.
Fig. 4 shows the sequences of nucleic acids used in the Examples (Examples of the invention and Comparative Examples).
Fig. 5 shows the efficiency of integration of retrovirus nucleic acid into a nucleic acid having the sequence as shown in Fig. 4.

### Best Mode for Carrying Out the Invention

### Nucleic acid

A nucleic acid of the present invention has an activity as a target of retrovirus integration and has a substantial palindromic sequence comprising:
a motif sequence represented by the following general formula (1) :

   5' -α₁-α₂-...-αₙ-x-βₙ-...-β₂-β₁-3'
wherein α₁ to αₙ each represent a sequence consisting of contiguous 4 to 7 bases in 5'-CACAGTG-3' or 5'-CACTGTG-3', X represents an arbitrary sequence consisting of 0 to 10 bases, β₁ to βₙ each represent a sequence consisting of 4 to 7 bases substantially complementary, in the opposite direction, to the sequence α₁ to αₙ, respectively, n represents an integer of 1 or more, and an arbitrary sequence consisting of one to a few bases may be present between the adjacent sequences among the sequences α₁ to αₙ and β₁ to βₙ; and
upstream and downstream sequences adjacent to the motif sequence, each of which has a length of 2 bases or more.

That is, the nucleic acid of the present invention has a substantial palindromic sequence capable of potentially taking a hairpin structure or a hairpin loop structure, and when such a structure is taken, base pairs of a part of:

5'-CACAGTG-3' ::::: 3'-GTGTCAC-5'

is formed in a stem by the sequences α and β. The sequence of these 7 base pairs coincides with a sequence called an immunoglobulin gene recombination signal sequence (RSS). Accordingly, this sequence may be hereinafter referred to as the "RSS element".

The sequences α and β in the nucleic acid of the present invention are substantially complementary in the opposite direction and can form essentially base pairs in a potential hairpin or hairpin loop structure; specifically, 50% or more of bases of a shorter sequence in apair of corresponding sequences α and β may form base pairs. For example, when each of the corresponding sequences α and β has 4 bases in length, 2 or more base pairs may be formed. Also, when the sequence α is 5 bases in length and its corresponding sequence β is 4 bases in length, the sequences α and β are "substantially complementary" if 2 base pairs are formed.

In the nucleic acid of the present invention, at least one pair, or two or more pairs, of the sequences α and β may be present in the motif sequence. When one pair is present, the motif sequence is 5' -α₁-X-β₁-3' . When two or more pairs are present, the motif sequence is, for example, 5'-α₁-α₂-X-β₂-β₁-3' (β₁ and α₁, and β₂ and α₂, are substantially complementary to each other, that is, in the opposite direction) . More generally, it may be arranged to form a palindromic sequence as a whole, as represented by the formula (1) : 5'-α₁-α₂-...-αₙ-x-βₙ-...-β₂-β₁-3'.

When the palindromic sequence in the nucleic acid of the invention is represented by this formula, n is an integer of 1 or more, generally in the range of 1 to 10, advantageously 1 to 6. Each of the sequences α or the sequences β may not be the same, and an arbitrary sequence consisting of one to a few bases may be present therebetween. Preferably, this arbitrary sequence can form base pairs with the corresponding sequence at the corresponding position in a potential hairpin structure.

In the nucleic acid of the present invention, the sequence X may or may not be present, and the sequence X when present may contain a sequence capable of forming base pairs inside of the sequence to form a stem, a sequence capable of forming a loop, or both.

The sequence X is preferably 0 to 10 bases, more preferably 0 to 9 bases, and may be composed of 0 to 6 bases for example.

Each of the upstream and downstream sequences adjacent to the motif sequence has a length of 2 bases or more, and such sequences also constitute a part of the substantial palindromic sequence of the nucleic acid of the present invention. Accordingly, when the nucleic acid of the present invention forms a hairpin or hairpin loop structure, base pairs formed by the upstream and downstream sequences adjacent to the motif sequence will occur in addition to the two or more base pairs that can be formed by the motif sequence. For example, in the nucleic acid of the invention obtained on the basis of a naturally occurring sequence, TCC or TTC is often present in the upstream adjacent sequence, while GGA or GAA is often present in the downstream adjacent sequence, and these sequences when present can form additional 3 base pairs.

Each of the sequences adjacent to the motif sequence is composed of preferably 4 or more bases, more preferably 6 or more bases, still more preferably 8 or more bases, and most preferably 10 or more bases. In particular, each of the upstream and downstream sequences adjacent to the motif sequence is composed advantageously of 10 to 50 bases.

The upstream and downstream sequences adjacent to the motif sequence may not be perfect palindromic sequences or sequences complementary in the opposite direction, composed of the same number of bases with no mismatch, and may be different in length. However, the difference in the number of bases composing both sequences is preferably small for allowing the nucleic acid of the present invention to have a substantial palindromic sequence; for example, the number of different bases in the sequence consisting of 20 bases is 10 or less, preferably 8 or less, more preferably 5 or less, still more preferably 3 or less, and most preferably the upstream and downstream sequences have the same length.

The minimum length of the motif sequence in the nucleic acid of the present invention is 8 bases (that is, each of the sequences α and β is 4 bases and X = 0) . Each of the upstream and downstream sequences adjacent to the motif sequence may be as short as 2 bases, but preferably 6 bases or more. Accordingly, the length of the palindromic sequence in the nucleic acid of the invention is 12 bases at the minimum, preferably 28 or more bases, for example 28 to 124 bases, and more preferably 36 to 100 bases. The number of base pairs contained in the palindromic sequence in the nucleic acid of the present invention is 3 base pairs at the minimum, preferably 7 or more base pairs, for example 7 to 62 base pairs, and more preferably 18 or more base pairs, for example 18 to 50 base pairs. Alternatively, preferably 50% or more, more preferably 60% or more, still more preferably 70% or more, of a half of the total number of the palindromic sequence excluding the sequence X form base pairs.

The nucleic acid of the present invention may be DNA or RNA insofar as it has the sequence as described above. Unless otherwise specified, each base in the nucleic acid of the present inventionmaybe abase analogue or amodifiedbase, or the nucleic acid itself may be modified or altered, insofar as the activity as a target of retrovirus integration described later is not destroyed. Modification and alteration as well as analogues of such bases or nucleic acid are known in the art.

The nucleic acid of the present invention, by having the sequence as described above, can take a hairpin or hairpin loop structure, regardless of whether a hairpin or hairpin loop structure is actually taken or not under specific conditions. Preferably, a calculated change in the free energy of the nucleic acid of the present invention in forming a hairpin structure has a large negative absolute value. When the nucleic acid of the present invention has formed a hairpin structure, the double-stranded chain preferably has a calculated change in free energy, ΔG = not higher than -5.0 kcal/mol. This change in free energy is preferably ΔG = not higher than -8.0 kcal/mol, more preferably ΔG = not higher than -12.0 kcal/mol, and most preferably ΔG = not higher than -15.0 kcal/mol. Particularly, ΔG = -18.0 to -20 kcal/mol is convenient. Calculation of free energy can be carried out according to Michael Zuker (2003), "Mfold web server for nucleic acid folding and hybridization prediction" (Nucleic Acids Res. 31 (13), 3406-15), on the basis of Santa Lucia John Jr. (1998), "A uniformed view of polymer, dumbbell, and oligonucleotide DNA nearest-neighbor thermodynamics" (Proc. Natl. Acad. Sci. USA 95, 1460-65).

One specific example of the motif sequence in the nucleic acid of the present invention is the sequence (5'-CACTGACATTATCACA-3') set forth in SEQ ID NO: 1. The underlined portions correspond to the sequences α and β, respectively.

One example of the motif sequence having 2 pairs of the sequences α and β is the sequence (5'-CAGTGGACACTGACATTATCACACTCCACT-3') set forth in SEQ ID NO: 2. The underlined portions correspond respectively to α₁, α₂, β₂ and β₁ in the order from the 5'-end in the formula (1). In the sequence set forth in SEQ ID NO: 2, "TG" in the base numbers 11 to 12, and "CA" in the base numbers 20 to 21, and "AGTG" in the base numbers 2 to 5 and "CACT" in the base numbers 27 to 30 are respectively palindromic sequences, and can potentially form base pairs to form a stem in a hairpin structure.

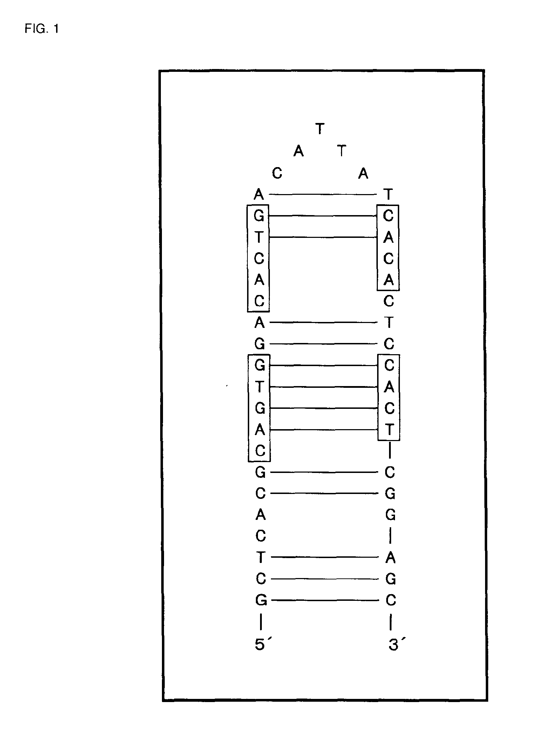

One example of the palindromic sequence in the nucleic acid of the present invention containing the motif sequence set forth in SEQ ID NO: 2 is the sequence (5'-GCTCACGCAGTGGACACTGACATTATCACACTCCACTCGGAGC-3') set forth in SEQ ID NO: 3. The hairpin structure potentially formed by the nucleic acid having this sequence is shown in Fig. 1.

The sequence (5'-CGCAGTGGACACTGACATTATCACACTCCACTCCG-3') set forth in SEQ IDNO: 5 is another example of the palindromic sequence containing the motif sequence having two pairs of the sequences α and β in the formula (1). The underlined portions correspond to α₁, α₂, β₂ and β₁ in the order from the 5' -end, similarly as described above.

The sequence (5'-ACACTGACATTATCACACT-3') set forth in SEQ ID NO: 7 is an example which contains the α-X-β motif sequence set forth in SEQ ID NO: 1, wherein the upstream adjacent sequence is one base (A), the downstream adjacent sequence is two bases (CT), and the base pair formed between the adjacent sequences is only one pair.

These are sequences designed on the basis of a sequence corresponding to the base numbers 4420 to 4520 in GENBANK Registration No. AF049104 (Mus musculus signal transducer and transcription activator 5a (Stat5a) gene, partial cds.).

The sequence of the nucleic acid having the palindromic sequence of the present invention may be a naturally occurring sequence as described above or an artificial sequence modified on the basis of the naturally occurring sequence, or may be a completely artificially designed sequence. For example, the sequence set forth in SEQ ID NO: 9 (5'-CACAGTGCACAGTGGACATTATCACAGTGCACAGTG-3') is an example of an artificially produced motif sequence having two pairs of the sequences α and β in the formula (1). The underlined portions are 2 repeated sequences having the 7 bases of RSS element and correspond to α₁ and α₂, and β₂ and β₁ in the order from the 5' -end of the formula (1).

The sequence set forth in SEQ ID NO: 10 (5'-GCTCCACAGTGCACAGTGGACATTATCACAGTGCACAGTGGAGC-3') is still another example of the palindromic sequence, containing the motif sequence set forth in SEQ ID NO: 9, wherein the upstream and downstream adjacent sequences each consisting of 4 bases can form 4 base pairs.

These artificial sequences have an activity as a target of retrovirus integration, which have efficiency equal to or higher than that of a sequence based on the naturally occurring sequence.

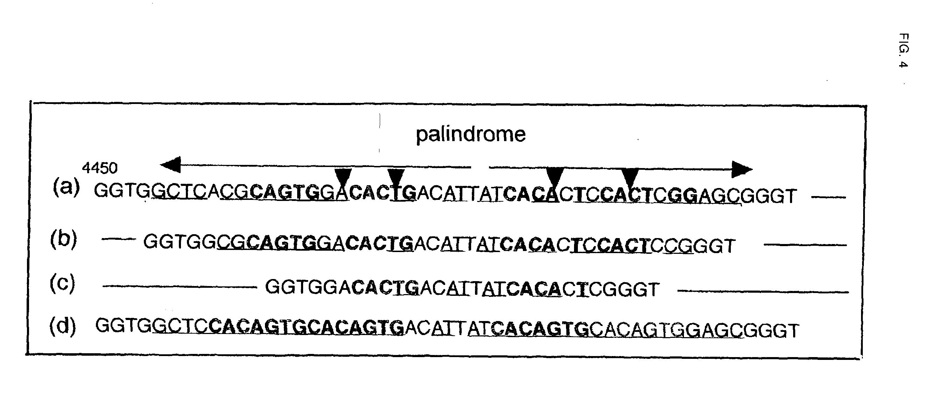

The sequences set forth in SEQ ID NO: 4, SEQ ID NO: 6, SEQ ID NO: 8 and SEQ ID NO: 11 are sequences of the full-length inserts (used in the Examples) including palindromic sequences set forth in SEQ ID NO: 3, SEQ ID NO: 5, SEQ ID NO: 7 and SEQ ID NO: 10, respectively (see Fig. 4).

Sequences capable of hybridizing with the motif sequence set forth in any of SEQ IDNOS : 1, 2 and 9 under stringent conditions and satisfying the conditions in the formula (1) are also examples of the motif sequence in the nucleic acid of the present invention. Similarly,sequences capable of hybridizing with the palindromic sequence set forth in any of SEQ ID NOS: 3, 5 and 10 under stringent conditions and satisfying the conditions of the palindromic sequence described above are also examples of the palindromic sequence in the nucleic acid of the present invention. The stringent conditions are those in 0.1×SSC buffer at 55°C.

The nucleic acid of the present invention can be synthesized by chemical or biochemical methods known in the art. For example, direct synthesis with a DNA synthesizer, PCR and/or a method of using a cloning vector can be used appropriately alone or in combination.

The nucleic acid of the present invention can bind to an integrase to inhibit integration of a retrovirus genome. The occurrence of binding to the integrase and the presence of the integration target activitycanbe confirmed by methods described later. For example, the nucleic acid of the invention having the sequence set forth in SEQ ID NO: 4 is integrated at an efficiency of about 80% or more when tested by a method described later. When the same sequence modified such that TG and/or CA was removed from the motif sequence is tested in the same manner, it has been found that the efficiency of integration of a viral gene into this sequence is reduced to 10% or less.

The activity as a target of retrovirus integration of the nucleic acid of the present invention (integration efficiency), when determined by examining at least 10 clones by the method described later, is preferably 40% or more (that is, integration occurs in 4 or more clones), more preferably 50% or more, still more preferably 60% or more, and most preferably 70% or more.

The nucleic acid of the present invention can be contained in a vector. The vector can be produced, for example, by integrating the nucleic acid of the present invention into a cloning site of a commercial cloning vector selected depending on the object (large-scale expression, medical purposes, and so on) . Designing and construction of such a vector are known to those skilled in the art.

### Antiviral agent

For prophylaxis and therapy of diseases attributable to a retrovirus, the nucleic acid of the present invention, either as such or combined with other ingredients, can be used as an antiviral virus, particularly a decoy-type drug or an antisense drug.

The term "decoy" or "decoy-type drug" refers to a nucleic acid having a sequence identical with or similar to that of the site on a chromosome to which an integrase can bind. The decoy having the identical sequence includes one consisting of the nucleic acid of the present invention. The decoy having a similar sequence includes one wherein one base in the sequence α and/or β in the motif sequence is substituted such that the substituted base does not form a new base pair with its surrounding sequence, preferably one wherein G in TG or GT is substituted with A or T, and C in CA or AC is substituted with A or T.

Upon administration, the decoy competes with the chromosomal site for binding to an integrase and binds to the integrase, thereby inhibiting the binding of the integrase to the chromosomal site and inhibiting retrovirus integration.

The antiviral agent of the present invention comprises at least one nucleic acid of the present invention or a vector containing the nucleic acid of the present invention, and a pharmaceutically acceptable carrier. When the nucleic acid of the present invention is used in the form of a vector, a vector wherein the 5'-side (A) and 3'-side (B) of a cloning site into which the nucleic acid of the present invention is inserted are substantially complementary in the opposite direction, for example, a vector having a cloning site 5'-GAANNNCCTTAAGGNNNTTC-3' wherein the nucleic acid of the present invention is cloned between T and A in TTAA, and NNN sequences at both ends are complementary, is preferable because the cloning site itself contributes to the formation of potential-hairpin-structure stem as a part of the palindromic sequence. The pharmaceutically acceptable carrier includes, for example, physiological saline, water, a buffer, dextrose, and so on.

The antiviral agent of the present invention can contain other ingredients known in the art, for example, ingredients such as a stabilizer, an excipient, a diluent and a carrier and other antiviral drugs.

The antiviral agent of the present invention can be administered through an oral or parenteral route. Specific examples of the route include, but are not limited to, oral, subcutaneous, intramuscular, intravenous, intraperitoneal, transdermal and transmucosal routes.

The antiviral agent of the present invention can be formulated in any arbitrary dosage form known in the technical field of pharmaceutical preparation, depending on the route of administration. Examples of the dosage form include, but are not limited to, tablets, capsules, granules, syrups, liquids, suspensions, gels and liposomes. These formulations can be produced by methods known in the art.

The antiviral agent of the present invention contains the nucleic acid of the present invention at an effective amount (pharmaceutically effective amount) to achieve its medical purposes. The effective amount is generally about 0.1 to 1 mg/kg, and its specific amount is determined by using a suitable animal model and the like. by methods known in the art.

The accurate dosage and administration schedule can be determined individually depending on the circumstances of an individual to which the agent is administered (for example, age, body weight, sex, the severity of infection or disease), another drug administered or a therapeutic method in combination with the agent, and the duration (half-life) of a specific formulation in the living body.

The nucleic acid of the present invention acts via a general mechanism of action utilizing the fundamental nature of various retroviruses and thus acts on essentially all retroviruses equally. The nucleic acid of the present invention is particularly effective against retroviruses living on hosts such as mammals including a mouse, cat, monkey, human, bovine, swine and so on., for example, against human immunodeficiency virus (HIV-1, HIV-2), adult T-cell leukemia virus (HTLV-I), hairy cell leukemia virus (HTLV-II), leukemia viruses in animals other than humans, sarcoma virus, mammary tumor virus, simian immunodeficiency virus (SIV), visna virus, equine infectious anemia virus, and foamy virus.

### Method of testing the activity as a target of retrovirus integration

The method of the present invention comprises at least the steps of: (1) allowing double-stranded nucleic acids having protrusions derived respectively from the 5'- and 3'-ends of a retrovirus genome LTR sequence or single-stranded nucleic acids capable of forming them, an integrase, and a cyclic nucleic acid containing a target sequence as a subject of examination to be simultaneouslypresent; and (2) detecting the presence or absence of the integration of the retrovirus genome-derived sequence into the target sequence.

In the method of the present invention, the target sequence is in a cyclic form and is preferably linked with a suitable vector. The target sequence preferably has a length of 100 or more base pairs. In the conventional integration assay, a linear oligonucleotide usually having a length of 20 to 30 base pairs has been used as a target sequence. In contrast, in the method of the present invention, a relatively long target sequence in a cyclic form is used, whereby self-cyclization of the target sequence can be prevented and the occurrence of the binding of the target sequence to the integrase can be judged more clearly.

In the method of the present invention, two nucleic acids derived respectively from the 5' - and 3' -ends of the retrovirus genome LTR sequence are used as the substrate nucleic acid. In the conventional method a nucleic acid of either the 5' - or 3' -end has been used. In contrast, in the method of the present invention, both nucleic acids derived from the 5' - and 3' -ends of the retrovirus genome LTR sequence are used, whereby an integrase dimer or tetramer is formed between an integrase and the four single-stranded chains contained in the two kinds of double-stranded chains in the substrate nucleic acid, and acts advantageously for integration reaction to improve the detection sensitivity of the integration reaction.

In the step (1) described above, preferably, two nucleic acids derived respectively from the 5'- and 3'-ends of the retrovirus genome LTR sequence are simultaneously combined with other reaction components. This step can be carried out in two substeps: substep (a) in which nucleic acids derived from the 5' - and 3' -ends of the retrovirus genome LTR sequence are combined with an integrase to form substrate nucleic acid/integrase complexes, and substep (b) in which the complexes are combined with a cyclic nucleic acid containing a target sequence. For example, the step (1) can be carried out by reacting nucleic acids derived from the 5' - and 3' -ends of the retrovirus genome LTR sequence separately with an integrase to form integrase complexes respectively, and then combining the both complexes simultaneously with the cyclic nucleic acid for reaction. By previously forming the integrase/substrate nucleic acid complexes in this manner, the integration occurs more easily to improve detection sensitivity of integration with a poor frequency.

Each of nucleic acids derived from LTR preferably has 50 or more bases, more preferably 60 or more bases, for example, 50 to 100 bases in length.

The target sequence preferably has a length of 100 or more bases. When the cyclic nucleic acid containing a target sequence is in a form inserted into a cloning site of a cloning vector and the cloning site has a sequence capable of forming a stem portion of a hairpin structure, the target sequence maybe shorter by about 10 to 20 base pairs, and may be 80 or more base pairs. Accordingly, the length of the target sequence is generally preferably 80 or more base pairs, more preferably 100 to 400 base pairs, still more preferably 150 to 400 base pairs, and most preferably 200 to 400 base pairs.

At the end of the substrate nucleic acid, there is preferably a protrusion of 2 bases from the 5' -end of (+) chain/(-) chain. For example, 5' -AA is preferably protruded in the case of MuLV, and 5' -AC is preferably protruded in the case of HIV. More preferably, in each of the single-stranded nucleic acids, the protruded 2 bases are followed by TG, and the 3' -end is CA.

Detection of occurrence of the integration can be carried out by any methods known in the art. Generally, the nucleic acid is amplified by PCR or the like, and then, the sequence of its reaction product is analyzed to specify the presence or absence of insertion as well as a site of insertion.

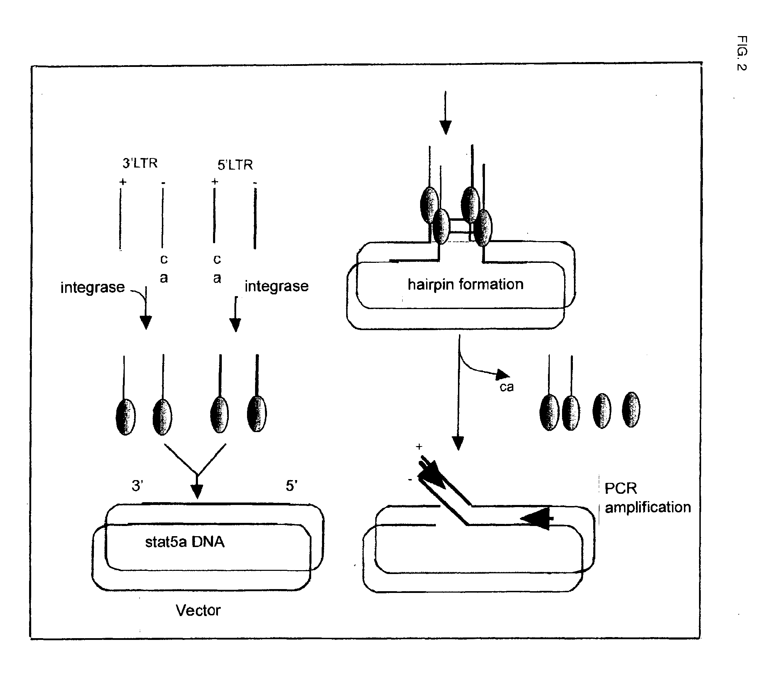

Fig. 2 shows an outline of one embodiment of the method of the present invention. In this case, four kinds of single-stranded nucleic acids derived from 3' LTR and 5' LTR are combined with an integrase to form substrate nucleic acid/integrase complexes (integrase complex intermediates) which are then combined with a vector containing a target sequence ("stat5a DNA"). The target sequence forms a hairpin, and the substrate nucleic acid is inserted into the target sequence. Thereafter, primers having a sequence near the target sequence or in the substrate sequence are used to amplify the target sequence portion by PCR to detect the presence or absence of insertion.

### Examples

Hereinafter, the present invention will be described in more detail by reference to specific examples.

### 1. Test of the activity as a target of retrovirus integration using MuLV (mouse leukemia virus; AKV-1) integrase

### 1) Preparation of target DNAs

A portion (base numbers 31 to 81 in SEQ ID NO: 12) of the nucleotide sequence of murine transcriptional factor stat5a gene with GENEBANK Registration No. AF049104 was subcloned in a TOPO-pCR2.1 vector (Product No. 45-0641, Invitrogen, CA) and designated Svi1. Separately, a similar clone was constructed except that the nucleotide sequence of the stat5a gene in the insert was modified by replacing C in base number 52 in SEQ ID NO: 12 with G, and designated MutSvi-1. Similarly, a clone in which the nucleotide sequence of the stat5a gene in the insert was modified by replacing the same C with A was constructed and designated MutSvi-2. These were used as target DNAs.

### 2) Preparation of recombinant integrase

A vector expressing MuLV integrase was prepared by ligating an integrase gene of AKV-1 (SEQ ID NO: 13, corresponding to the base numbers 4626 to 5840 of GENBANK, MLOCG [JO1998] AKV murine leukemia virus, complete proviral genome., murine leukemia virus) to a pTrcHis2-TOPO vector (Version G, Catalogue No. K4400-40, Invitrogen).

The resulting vector was expressed in *Escherichia coli* by culturing at 37°C for 5 hours in the presence of 1mM IPTG, and MuLV integrase was recovered from the culture fluid and purified by using a ProBond column (Invitrogen) according to a manual attached to the product.

### 3) Binding of the integrase to the end (s) of retrovirus genome

75 ng of AKV-1 U5LTR-derived DNAs [SEQ ID NO: 14, (+)TGAAAGACCCCTTCATAAGGCTTAGCCAGCTAAC TGCAGTAACGCCATTTTGCAAGGCATGGGAAAATACCAGAGCTGA and SEQ ID NO: 15, (-) AATCAGCTCTGGTATTTTCCCATGCCTTGCAAAATGGCGTTACTGCAGTTAGCTGG CT AAGCCTTATGAAGGGGTCTTTCA] were incubated at 30°C for 1 hour with 500 ng of recombinant MuLV-1 integrase in 10 mL of reaction buffer (25 mM MnCl₂, 9% (V/V) glycerol, 80mMpotassiumglutamate, 10 mM mercaptoethanol, 10% (V/V) DMSO, 35 mM MOPS (pH 7.2)). The AKV-1 U5-derived DNAs, when forming the double-stranded chain, had a protrusion of two bases (AA) at the 5' -end of the (-) chain (or a recession of 2 bases at the (+) chain).

Similarly, 75 ng of AKV-1 U3LTR-derived DNAs [SEQ ID NO: 16, (+) AAATCGTGGTCTCGCTGATCCTTGGGAGGGTCTCCTCAGAGTGATTGACTGCCCAG CCTGGGGGTCTTTCA and SEQ ID NO: 17, (-) TGAAAGACCCCCAGGCTGGGCAGTCAATCACTCTGAGGAGACCCTCCCAAGGATCA GCGAGACCACGAT] were incubated at 30°C for 1 hour with the recombinant MuLV-1 integrase. The AKV-1 U3-derived DNAs, when forming the double-stranded chain had a protrusion of two bases (AA) at the 5'-end of the (+) chain (or a recession of 2 bases at the (-) chain).

### 4) Reaction of the target DNA with the retrovirus genome end (s) / integrase complexes

The U5LTR/integrase complex and U3LTR/integrase complex prepared in 3) above were combined with 200 ng of each of the target DNAs prepared in 1) above and incubated at 30°C for 1 hour.

### 5) PCR amplification of virus/target DNA insertion site

After the incubation was finished, PCR amplification of the above reaction product (5 µL) was carried out by using any one of AKV-1 U3LTR genome primer "MuLV U3-Stat5alF" (forward) (SEQ ID NO: 18, TCC TCCGATAGA CTGAGT CG), "MuLV U3-Stat5a2F" (nested forward) (SEQ ID NO: 19, TTCATTCACACTCCACTC GG) , U5LTR primer "MuLV U5-Stat5a1F" (forward) (SEQ ID NO: 20, TTAGCACCAGAGCGACTAGG) and "MuLV U5-Stat5a2F" (forward) (SEQ ID NO: 21, CAGGAAACAGCTATGACCATG), and a TOPO vector primer (reverse) (SEQ ID NO: 22, CGTCTGTTGTGTGACTCTGG).

The temperature cycle consisted of incubation at 94°C for 2 minutes, then 35 cycles of 95°C for 40 seconds, 58°C for 40 seconds and 72°C for 1 minute, and finally 72°C for 5 minutes.

### 6) Nucleotide sequence analysis

The PCR product obtained in the step described above was analyzed for its nucleotide sequence by subcloning it in the TOPO-pCR2.1 vector.

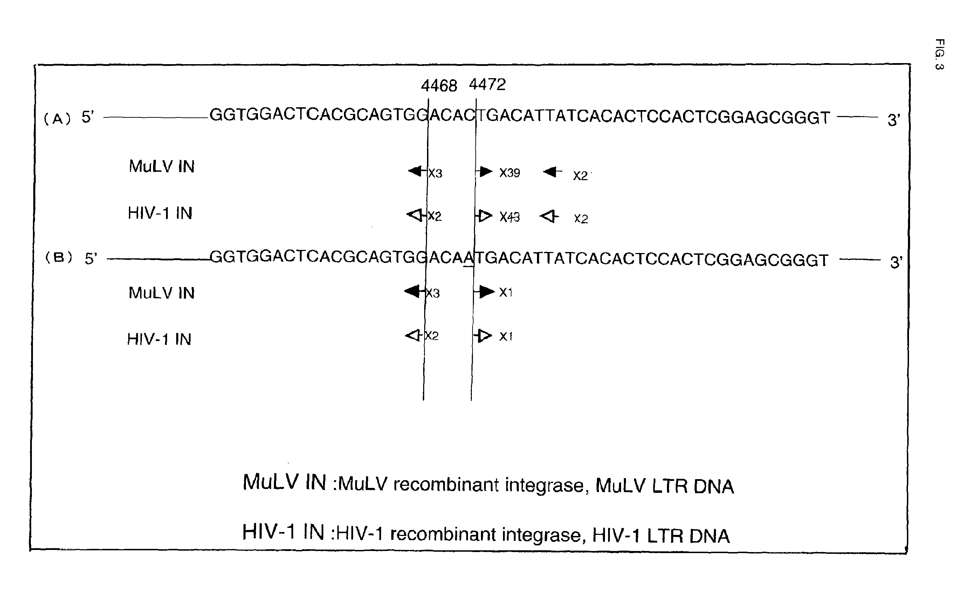

The results are shown in Fig. 3. The DNA sequence in the upper (A) shows a sequence containing an Svi1 integration site, and the DNA sequence in the lower (B) shows a sequence of MutSvi-2 . The underlined portion of the lower DNA sequence shows a substitutedportion. In Fig. 3, the base of each arrow indicates an integration site. The direction of the arrows represents the direction of transcription of virus genome; in the case of the right-pointing arrow, the direction of transcription agrees with that of the target sequence (Stat5a), and in the case of the left-pointing arrow, the direction of transcription is in the opposite direction. The number after "X" indicates the number of clones whose integration was recognized at that position.

When Svi1 was used as the target (Fig. 3, (A) , "MuLV IN") , insertion occurred in at least 90% of the examined clones, and it was found that the insertion occurred not at random sites but at specific sites (positions designated "4468" and "4472") with high frequencies. On the other hand, when MutSvi-2 (Fig. 3, (B), "MuLV IN") was used as the target, insertion hardly occurred at the modifiedportion. MutSvi-1 gave a similar result (data on MutSvi-1 are not shown).

### 2. Test of the activity as a target of retrovirus integration using HIV-1 integrase

### 1) Target DNA and recombinant integrase

The target DNAs used were the same as that for the above-mentioned MuLV integrase. As the recombinant HIV-1 integrase, a commercial product was used (Catalog No. H6003-15, "HIV-1 pol p31, Met 737-1003, SF-2, Recombinant (Integrase, Human), US Biological Ltd.).

### 2) Binding of the integrase to the end (s) of retrovirus genome

75 ng of AKV-1 U5LTR-derived DNAs [SEQ ID NO: 23, (+) TGTGTGCCCGTCTGTTGTGTGACTCTGGTAACTAGAGATCCTCAGACCTTTTTGGTAGT GTGGAAAATCTCTAGCAC and SEQ ID NO: 24, (-)ACTGCTAGAGATTTTCCACACTACCAAAAAGGGTCTGAGGGATCTCTAGTTACCAG AGTCACACAACAGACGGGCACAC] were incubated at 30°C for 1 hour with 500 ng of the recombinant HIV-1 integrase in 10 mL of reaction buffer.

Similarly, 75 ng of HIV-1 U3LTR-derived DNAs [SEQ ID NO: 25, (+) ACTGGAAGGGTTAATTTACTCCAAGCAAAGGCAAGATATCCTTGATTTGTGGGTCT ATAACACACAAGGCTACTTCCCAG and SEQ ID NO: 26, (-) CTGGGAAGTAGCCTTGTGTGTTATAGACCCACAAATCAAGGATATCTTGCCTTTGC TTGGA GTAAATTAAC CCTTCCA] were incubated at 30°C for 1 hour with the recombinant HIV-1 integrase. The nucleotide sequence is based on 99ZACM9 (GENEBANK Accession No. AF411967) from South Africa.

### 3) Reaction of the target DNA with the retrovirus genome end(s)/integrase complexes

The U5LTR/integrase complex and the U3LTR/integrase complex prepared in 2) above were combined with 200 ng of each of the target DNAs prepared in 1) above and incubated at 30°C for 1 hour.

### 4) PCR amplification of virus/target DNA insertion site

After the incubation was finished, PCR amplification of the above reaction product (5 µL) was carried out in the same manner as in the above MuLV. The primers used were a primer set of any one of HIV-1U5LTR genome primer "HIV"-1U5LTR" (reverse) (SEQ ID NO: 27, CGTCTGTTGTGTGACTCTGG), HIV-1 U3LTR genome primer "HIV-1 U3LTR" (reverse) (SEQ ID NO: 28, GGGAAGTAGCCTTGTGTGTTATAG), "HIV-Stat5a1F" (forward) (SEQ ID NO: 29, TTAGCACCAGAGCGACTAGG) and "HIV-Stat5a2F" (forward) (SEQ ID NO: 30, CAGGGAAACAGCTATGACCATG), and a TOPO vector primer.

### 6) Nucleotide sequence analysis

The PCR product obtained in the step described above was analyzed for its nucleotide sequence in the same manner as in the above-mentioned MuLV by subcloning it in the TOPO-pCR2.1 vector.

The result is shown in "HIV-1 IN" in Fig. 3. In HIV-1, as in the case of MuLV, integration occurred highly frequently at specific positions, and when a base in the motif sequence was modified, the result of significant reduction in the frequency of integration was obtained.

### 3. Measurement of the activity as a target of retrovirus integration of various sequences

The frequency at which integration into the target nucleic acid occurred by using MuLV integrase or HIV integrase in the same manner as described for MuLV was examined by using, as the target DNA, a cyclic DNA obtained by subcloning an insert having each of the sequences (a) to (d) shown in Fig. 4 into the TOPO-pCR2.1 vector in the same manner as in the above test. Nucleotide sequences of 20 clones selected at random for each case were analyzed.

The sequences (a) to (d) are shown in SEQ ID NOS: 4, 6, 8 and 11, respectively. Each of these sequences contains the palindromic sequence of the present invention. However, the sequence (c) has shorter upstream and downstream sequences adjacent to the motif sequence, and nucleic acid having the sequence (c) is a comparative example not satisfying the requirements of the nucleic acid of the present invention.

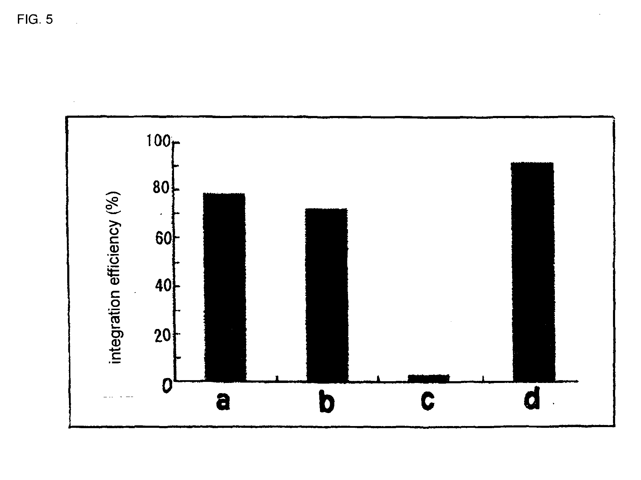

The results are shown in Fig. 5. When the nucleic acid having the sequence (a), (b) or (d) is used as the target nucleic acid, integration occurred at 70% or more of the clones whose sequences had been examined. On the other hand, when the nucleic acid having the sequence (c) was used as the target nucleic acid, clones in which integration had occurred were observed at a low frequency.

## Claims

1. A nucleic acid having an activity as a target of retrovirus integration, which has a substantial palindromic sequence comprising:
a motif sequence: 5'-α₁-α₂-...-αₙ-X-βₙ-...-β₂-β₁-3' wherein α₁ to αₙ each represent a sequence consisting of contiguous 4 to 7 bases in 5'-CACAGTG-3' or 5'-CACTGTG-3', X represents an arbitrary sequence consisting of 0 to 10 bases, β₁ to βₙ each represent a sequence consisting of 4 to 7 bases substantially complementary, in the opposite direction, to the sequence α₁ to αₙ, respectively, n represents an integer of 1 or more, and an arbitrary sequence consisting of one to a few bases may be present between the adjacent sequences among the sequences α₁ to αₙ and β₁ to βₙ; and
upstream and downstream sequences adjacent to the motif sequence, each of which has a length of 2 bases or more.

2. The nucleic acid according to claim 1, wherein each of the upstream and downstream sequences adjacent to the motif sequence consists of 4 or more bases.

3. The nucleic acid according to claim 1 or 2, wherein TCC or TTC is present in the upstream adjacent sequence, and GGA or GAA is present in the downstream adjacent sequence.

4. The nucleic acid according to any of claims 1 to 3, wherein the palindromic sequence is 36 to 100 bases in length.

5. The nucleic acid according to any of claims 1 to 4, which can bind to a retrovirus integrase.

6. The nucleic acid according to any of claims 1 to 5, wherein the motif sequence is a sequence set forth in any of SEQ ID NOS: 1, 2 and 9, or a sequence capable of hybridizing therewith under stringent conditions.

7. The nucleic acid according to any of claims 1 to 6, wherein the palindromic sequence is a sequence set forth in any of SEQ ID NOS: 3, 5 and 10, or a sequence capable of hybridizing therewith under stringent conditions.

8. The nucleic acid according to any of claims 1 to 7, which is in a form carried in a vector.

9. An antiviral agent comprising the nucleic acid according to any of claims 1 to 8.

10. The antiviral agent according to claim 9, which is a decoy-type drug or an antisense drug.

11. An antiviral agent comprising, as a decoy, a nucleic acid with a substitution of one base in any of one or more sequences α and/or β in the motif sequence according to any of claims 1 to 8.

12. A method of testing a nucleic acid for an activity as a target of retrovirus integration, comprising the steps of:
(1) allowing double-stranded nucleic acids having at the 5'-side thereof protrusions of 2 bases, which nucleic acids derived respectively from the 5'- and 3'-ends of a retrovirus genome LTR sequence, or single-stranded nucleic acids capable of forming such nucleic acids, an integrase, and a cyclic nucleic acid containing a target sequence as a subject of examination to be simultaneously present; and
(2) detecting the presence or absence of the integration of the retrovirus genome-derived sequence into the target sequence.

13. The method according to claim 12, wherein in the step (1), the nucleic acid derived from the 5' -end and the nucleic acid derived from the 3' -end of the retrovirus genome LTR sequence are simultaneously combined with the other reaction components.

14. The method according to claim 12, wherein in the step (1), the nucleic acid derived from the 5'-end and the nucleic acid derived from the 3' -end of the retrovirus genome LTR sequence are reacted with the integrase separately to form integrase complexes, and then these integrase complexes are simultaneously combined and reacted with the cyclic nucleic acid.

15. The method according to any of claims 12 to 14, wherein the target sequence is at least 100 base pairs in length.

16. The method according to any of claims 12 to 15, wherein the detection of the presence or absence of the integration is carried out by nucleic acid amplification and subsequent sequence analysis.


[SEQUENCE LISTING]

<110>  Kyoto University

<120>  Nucleic acid having retroviral integration
target-activity

<130>  27282/004EP1

<150>  PCT/JP2005/015255
<151>  2005-08-23

<150>  JP 2004-244256
<151>  2004-08-24

<160>  30

<210>  1
<211>  16
<212>  DNA
<213>  Mus musculus

<400>  1
cactgacatt atcaca                                                    16

<210>  2
<211>  30
<212>  DNA
<213>  Mus musculus

<400> 2

cagtggacac tgacattatc acactccact 30

<210> 3

<211> 43

<212> DNA

<213> Mus musculus

<400> 3

gctcacgcag tggacactga cattatcaca ctccactcgg agc 43

<210> 4

<211> 51

<212> DNA

<213> Mus musculus

<400> 4

ggtggctcac gcagtggaca ctgacattat cacactccac tcggagcggg t 51

<210> 5

<211> 35

<212> DNA

<213> Mus musculus

<400>  5
cgcagtggac actgacatta tcacactcca ctccg                                35

<210>  6
<211>  43
<212>  DNA
<213>  Mus musculus

<400>  6
ggtggcgcag tggacactga cattatcaca ctccactccg ggt                       43

<210>  7
<211>  19
<212>  DNA
<213>  Mus musculus

<400>  7
acactgacat tatcacact                                                  19

<210>  8
<211>  29
<212>  DNA
<213>  Mus musculus

<400> 8

ggtggacact gacattatca cactcgggt 29

<210> 9

<211> 35

<212> DNA

<213> Artificial Sequence

<220>

<223> integration target motif

<400> 9

cacagtgcac agtgacatta tcacagtgca cagtg 35

<210> 10

<211> 43

<212> DNA

<213> Artificial Sequence

<220>

<223> parindrome sequence with integration target motif

<400> 10

gctccacagt gcacagtgac attatcacag tgcacagtgg agc 43

<210> 11

<211> 51

<212> DNA

<213> Artificial Sequence

<220>

<223> insertion sequence

<400> 11

ggtggctcca cagtgcacag tgacattatc acagtgcaca gtggagcggg t 51

<210> 12

<211> 100

<212> DNA

<213> Mus musculus

<400> 12

gacaagggat ccgtgcaggc ggcagccagg ggtggactca cgcagtggac

actgacatta 60

tcacactcca ctcggagcgg gtagattctg gagtgcagcc 100

<210> 13

<211> 1215

<212> DNA

<213> Murine leukemia virus

<220>
<223> integrase

<400> 13
atagaggatt caaccccata tacgcctgcc tatttccatt atactgaaac
agatctaaag     60

aaactcagag agcttggggc cacctataac cagagcaaag gatactgggt
cttccaaggc    120

aagccggtga tgcccgacca atttgtattt gaactgttag actcactcca
ccggctcacc    180

cacctcggct accagaaaat gaaagcactc cttgacagag gagaaagccc
ctactacatg    240

ctaaaccggg acaaaaccct ccaatatgtg gcagattcct gcacggtctg
tgcccaagta    300

aatgccagca aagctaaaat cggggcagga gtgagagtac gaggacatcg
gccaggctcc    360

cattgggaga tcgattttac agaagtcaag ccagggctgt atgggtacaa
gtacctcctg    420

gtattcgtgg acaccttctc tggttgggtg gaagcctttc caaccaagag
agaaacagca    480

agagtcgtgt ccaagaaatt gctggaagaa atattcccga gattcggaat
gccacaggta    540

ttgggatctg ataacgggcc tgccttcacc tcccaggtaa gtcagtcggt
ggccgattta    600

ctggggatcg attggaaatt acattgtgct tatagacccc agagttcagg
tcaggtagaa    660

agaatgaata gaaccatcaa ggagactcta actaaattaa cgcttgcagc
tggcactaga    720

gactgggtac tcctactccc cttagctctt taccgagccc ggaacactcc
gggcccccat    780

ggactgactc cgtatgaaat cttgtacggg gcgcccccgc cccttgttaa
cttccatgac    840

cccgacatgt cagaattaac taatagccca tctctccaag ctcacttaca
ggccctccaa    900

acggtgcagc gagaaatttg gaaaccactg gccgaggcct accgggacca
actagaccaa    960

ccagtgatac cacacccctt ccggattgga gactccgtgt gggtgcgccg
gcaccagacc    1020

aaaaacttag aacctcgctg gaagggaccc tacaccgtcc tactgaccac
ccccaccgct    1080

ctcaaggtag acggcatctc tgcatggata cacgccgccc acgtcaaggc
agcgaccacg    1140

cccccgataa aaccatcatg gagagtacaa cgctctcaaa accctttaaa
aatcaggtta    1200

acccgtgggg ccccc
1215

<210> 14
<211> 79
<212> DNA
<213> Murine leukemia virus

<220>
<223> U5LTR plus strand

<400> 14
tgaaagaccc cttcataagg cttagccagc taactgcagt aacgccattt
tgcaaggcat     60

gggaaaatac cagagctga 79

<210> 15

<211> 81

<212> DNA

<213> Murine leukemia virus

<220>

<223> U5LTR minus strand

<400> 15

aatcagctct ggtattttcc catgccttgc aaaatggcgt tactgcagtt

agctggctaa 60

gccttatgaa ggggtctttc a 81

<210> 16

<211> 71

<212> DNA

<213> Murine leukemia virus

<220>

<223> U3LTR plus strand

<400> 16

acatcgtggt ctcgctgatc cttgggaggg tctcctcaga gtgattgact

gcccagcctg     60

ggggtctttc a                                                    71

<210> 17

<211> 69

<212> DNA

<213> Murine leukemia virus

<220>

<223> U3LTR minus strand

<400> 17

tgaaagaccc ccaggctggg cagtcaatca ctctgaggag accctcccaa

ggatcagcga     60

gaccacgat                                                       69

<210> 18

<211> 20

<212> DNA

<213> Murine leukemia virus

<220>
<223> U3LTR primer

<400> 18
tcctccgata gactgagtcg                                                    20

<210> 19
<211> 20
<212> DNA
<213> Murine leukemia virus

<220>
<223> U3LTR primer

<400> 19
ttcattcaca ctccactcgg                                                    20

<210> 20
<211> 20
<212> DNA
<213> Murine leukemia virus

<220>
<223> U5LTR primer

<400> 20

ttagcaccag agcgactagg                                                    20

<210> 21

<211> 21

<212> DNA

<213> Murine leukemia virus

<220>

<223> U5LTR primer

<400> 21

caggaaacag ctatgaccat g                                                  21

<210> 22

<211> 20

<212> DNA

<213> Artificial Sequence

<220>

<223> TOPO vector primer

<400> 22

cgtctgttgt gtgactctgg                                                    20

<210> 23

<211> 77

<212> DNA

<213> Human immunodeficiency virus type 1

<220>

<223> U5LTR plus strand

<400> 23

tgtgtgcccg tctgttgtgt gactctggta actagagatc ctcagacctt

tttggtagtg     60

tggaaaatct ctagcac                                                    77

<210> 24

<211> 79

<212> DNA

<213> Human immunodeficiency virus type 1

<220>

<223> U5LTR minus strand

<400> 24

actgctagag attttccaca ctaccaaaaa gggtctgagg gatctctagt

taccagagtc     60

acacaacaga cgggcacac                                                    79

<210> 25
<211> 80
<212> DNA
<213> Human immunodeficiency virus type 1

<220>
<223> U3LTR plus strand

<400> 25
actggaaggg ttaatttact ccaagcaaag gcaagatatc cttgatttgt
gggtctataa      60

cacacaaggc tacttcccag                                                   80

<210> 26
<211> 78
<212> DNA
<213> Human immunodeficiency virus type 1

<220>
<223> U3LTR minus strand

<400> 26

ctgggaagta gccttgtgtg ttatagaccc acaaatcaag gatatcttgc

ctttgcttgg     60

agtaaattaa cccttcca                                                    78

<210> 27

<211> 20

<212> DNA

<213> Human immunodeficiency virus type 1

<220>

<223> U5LTR primer

<400> 27

cgtctgttgt gtgactctgg                                                  20

<210> 28

<211> 24

<212> DNA

<213> Human immunodeficiency virus type 1

<220>

<223> U3LTR primer

<400> 28

gggaagtagc cttgtgtgtt atag                                              24

<210> 29

<211> 20

<212> DNA

<213> Artificial Sequence

<220>

<223> HIV-Stat5a 1F primer

<400> 29

ttagcaccag agcgactagg                                                   20

<210> 30

<211> 22

<212> DNA

<213> Artificial Sequence

<220>

<223> HIV-Stat5a 2F primer

<400> 30

cagggaaaca gctatgacca tg                                                22